# EUROPEAN PATENT APPLICATION

(11) **EP 1 260 233 A1**
(43) Date of publication of application: **27.11.2002**
(21) Application number: 01908178.5
(22) Date of filing: 01.03.2001
(51) Int. Cl.: A61K 47/00, A61K 47/18, A61K 47/22, A61K 47/26, A61K 47/42, A61K 47/34, A61K 9/00, A61K 31/56, A61K 31/711, A61K 38/00, A61K 45/00

(54) **P-GLYCOPROTEIN MODIFIER-CONTAINING MEDICINAL COMPOSITIONS TO BE DELIVERED TO THE LARGE INTESTINE**

(30) Priority: 02.03.2000 JP 2000057630
(71) Applicant: HISAMITSU PHARMACEUTICAL CO. INC., Tosu-shi, Saga-ken 841-0017 (JP)
(72) Inventor: TANIDA, Norifumi, HISAMITSU PHARMACEUT. CO., INC., Tsukuba-shi, Ibaraki 305-0856 (JP); GOTO, Takeshi, HISAMITSUPHARMACEUTICAL CO., INC., Tsukuba-shi, Ibaraki 305-0856 (JP); KUROSAKI, Yuji, Joubou-gun, Okayama 716-1241 (JP)
(74) Representative: Cresswell, Thomas Anthony
(86) International application number: JP0101546
(87) International publication number: WO01064253

(57) **Abstract**

Novel medicinal compositions aiming at delivering a medicine to a specific site of the large intestine; and preparations for intestinal administration with the use of the same. Namely, medicinal compositions, whereby a medicine can be delivered to a specific site of the large intestine, characterized by containing a P-glycoprotein modifier; and preparations for intestinal administration with the use of the same.

## Description

### Technical Field

The present invention relates to a preparation for administering to the large intestine whereby a medicine is able to be delivered to a specific site of the large intestine and also relates to a medicinal composition thereof. The present invention relates to a preparation for administering to the large intestine which is characterized in being compounded with a P-glycoprotein modifier. More particularly, it relates to a preparation for administering to the large intestine containing a pharmacologically active substance which is to be a substrate for P-glycoprotein as a medicine, characterized in that, it is a medicinal composition to be delivered to the large intestine where P-glycoprotein modifier is compounded and also relates to a preparation for administering to the large intestine containing the same.

### Background of the Invention

The technical development in the art of delivering a medicine to the large intestine has been significant in recent years with an object for the production of an orally applicable preparation of peptides and also for the therapy of intestinal local diseases such as cancer of the colon and colitis. Conventional preparations for oral administration are usually disintegrated and eluted before reaching the large intestine and, therefore, the orally administered physiologically active polypeptide or oligonucleotide is easily decomposed by hydrolase in the small intestine. Further, when anti-inflammatory agent or anti-cancer agent is administered with an object of therapy of diseases of the large intestine such as cancer of the colon and colitis, such an agent is already absorbed at the small intestine in the case of the usual dosage form before it reaches the intestinal region which is a diseased site.

Under such backgrounds, various attempts have been made for a technical development in the art of delivering a medicine to the large intestine up to now. For example, there have been known an oral preparation, where the large intestine is a target for the release, by combining a polymer only dissolving at pH 5.5 or more with an insoluble polymer (European Patent Publication No. 49590) ; a solid orally administrable dosage form which is coated with an appropriate amount of an anionic polymer dissolving at pH 7.0 or more (trade name: Eudragit S; manufactured by Roehm) (International Application Laid-Open WO 83/00435), an oral preparation which is coated with an appropriate composition ratio of an anionic polymer dissolving at pH 7.0 or more (trade name: Eudragit S; manufactured by Roehm) and a methacrylate copolymer which is hardly soluble in water (trade name: Eudragit RS; manufactured by Roehm) (European Patent Publication No. 2225189), an osmotic pump preparation coated with an enteric coating polymer (Belgian Patent Publication No. 03502), an oral pharmaceutical formulation to be delivered to the large intestine where an inner layer dissolving at pH 7.0 or more is coated with a gelled polymer layer as an intermediate layer and further coated with a stomach-resisting outer layer dissolving at pH 5.5 or more thereon (Japanese Patent Publication No. 501411/1992), etc. In addition, several arts for delivering to the large intestine using a coating polymer for pharmaceutical excipients have been reported as well (International Application Laid-Open WO 90/13286, Japanese Patent Laid-Open No. 87169/1997 and International Application Laid-Open WO 95/28963). The inventors for this invention have also proposed an oral pharmaceutical formulation which is released at lower digestive tracts having a high specificity to the large intestine (International Application Laid-Open WO 94/10983 and Japanese Patent Laid-Open No. 152431/1998). That is a preparation which is characterized in comprising a double-coated structure where compressed and molded tablets or capsules wherein granules, powder or liquid are/is sealed are used as cores and are coated with an inner layer comprising a cationic copolymer and an outer layer comprising an anionic copolymer. The said preparation has a significantly good specificity to the large intestine and makes a surer and quicker release of the medicine targeting the large intestine possible.

As a result of such developments in the art, although it became possible to deliver the medicine to the large intestine in an unchanged state and to elute the medicine without delay, the property of targeting to the specific site of the large intestine has not yet been well achieved in the current state. For example, in the case of cancer of colon and colitis, the diseased part spreads all over the large intestine in some patients; while as for some other patients, the diseased part is limited to the descending colon; further as for some other patients, the situation is reversed being limited to the proximal colon. Thus, in order to provide a preparation meeting the needs of individual patients, it is necessary that the medicine is made more targetable to a specific site in the large intestine.

### Disclosure of the Invention

With regard to an art for the delivery of a medicine to the large intestine, although there have been made many developments as mentioned above, an art for the delivery of the medicine to a specific site of the large intestine has not been well established yet.

In the prior art, absorption in the upper area of the large intestine is large in the case of a medicine having a relatively quick absorption and, when the diseased site is located at the lower area of the large intestine, loss of the medicine is big causing a problem. Even tried to overcome such a problem by means of an improvement in dosage form though, it still remains the technical difficulties derived from the difference of the individual retention of the contents in the large intestine and the decrease of water content in the descending colon.

An object of the present invention is to provide a medicinal composition for a site-specific delivery to the large intestine which is most suitable as an orally administrable preparation being disintegrable in the large intestine and also to provide a preparation for administering to the large intestine containing the same.

### Brief Description of the Drawing

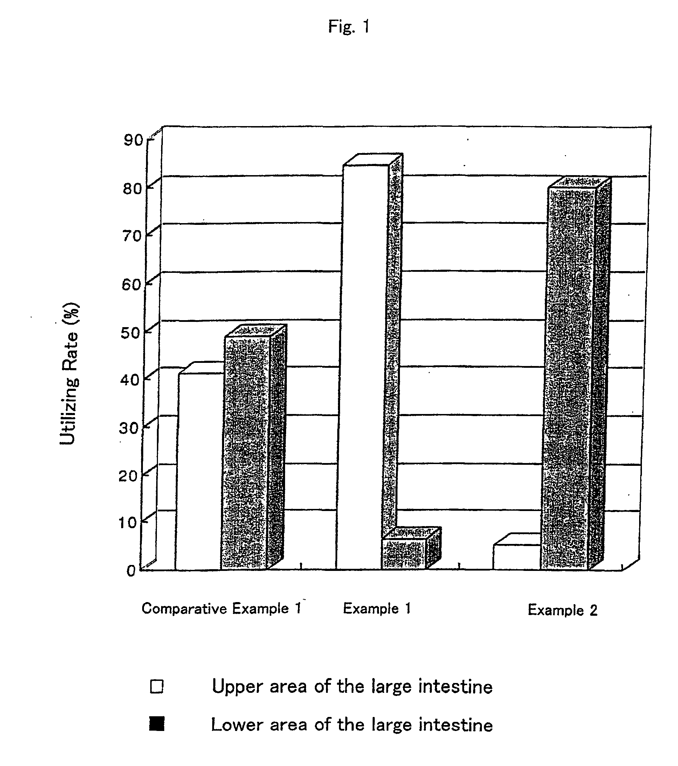

Fig. 1 is a graph where the utilizing rate of the medicine in the large intestine region in Test Example 1 is evaluated.

### Best Mode for Carrying Out the Invention

The present inventors have carried out an investigation for absorption of medicine from the large intestine using a steroid preparation which is one of the medicines for the therapy of colitis and found that, in the absorption of the medicine in the steroid preparation, there is a difference depending upon the site and the lower area of the large intestine shows a higher absorption than the upper area thereof. The present inventors have further found that such a difference in the absorption is dependent on a medicine discharging mechanism by P-glycoprotein which is locally present only at the upper area of the large intestine. In view of the above, the present inventors have found that, when an additive modifying the P-glycoprotein is compounded with a medicine which is able to be a substrate for P-glycoprotein, it is now possible to specifically deliver the medicine to the upper or the lower area of the large intestine.

Thus, the present invention relates to a medicinal composition to be delivered to the large intestine which is able to deliver the medicine to a specific site of the large intestine, characterized in that, a P-glycoprotein modifier is compounded therewith. More particularly, it relates to a medicinal composition to be delivered to the large intestine which is characterized in that, as a medicinal composition in a preparation for administering to the large intestine, a P-glycoprotein modifier is compounded with the medicine or, preferably, with the medicine which is able to be a substrate for P-glycoprotein.

The present invention also relates to a preparation for administering to the large intestine in which the above-mentioned medicinal composition to be delivered to the large intestine is contained in a medicine layer.

In an experiment for evaluation of the absorption of medicine from the large intestine in rats, the present inventors have carried out an investigation for the influence of known P-glycoprotein-acting medicine on the absorption of medicine and, as a result, they have found that, in the conventional preparations for administering to the large intestine comprising a steroid preparation only, their utilizing rate at the upper area of the large intestine is about 40% and that at the lower area of the large intestine is about 50% while, in the system where a P-glycoprotein inhibitor such as verapamil is compounded, the utilizing rate at the upper area of the large intestine increases to about 85% due to the contribution of inhibition of the medicine discharging system while the utilizing rate at the lower area of the large intestine becomes about 5% whereby a preparation which is selective to the upper area of the large intestine with a high selectivity can be obtained. On the other hand, it has been found that, in a system where a P-glycoprotein enhancer such as adenosine triphosphate is compounded, discharge of the medicine from the upper area of the large intestine is promoted and, in spite of administration of the medicine solution to the upper area of the large intestine, the utilizing rate at the upper area of the large intestine is as low as about 5% while the utilizing rate at the lower area of the large intestine reaches as high as about 80% whereby a specifically high utilizing rate is achieved.

As a result, it has been found that, when a P-glycoprotein modifier comprising a P-glycoprotein inhibitor or a P-glycoprotein enhancer is compounded with a preparation for administering to the large intestine, it is possible to prepare a medicinal composition to show a high utilizing rate at the specific site in the large intestine.

Accordingly, the first embodiment of the present invention, there is provided a medicinal composition where the medicine is delivered to the upper area of the large intestine. For such a purpose, there is provided a composition for a preparation to be delivered to the large intestine where a P-glycoprotein inhibitor is compounded with a medicine such as a pharmacologically active substance which is to be a P-glycoprotein substrate represented by a steroid preparation for the therapy of colitis. The case where the compounding ratio of the above-mentioned medicine to the P-glycoprotein inhibitor is from 1:0.01 to 1:10 is a preferred embodiment.

As the second embodiment of the present invention, there is provided a medicinal composition for delivering the medicine to the lower area of the large intestine. There is provided a composition for a preparation to be delivered to the large intestine where a P-glycoprotein enhancer is compounded with a medicine such as a pharmacologically active substance which is to be a P-glycoprotein substrate represented by a steroid preparation. The case where the compounding ratio of the above-mentioned medicine to the P-glycoprotein enhancer is from 1:0.01 to 1:10 is a preferred embodiment.

In the present invention, there is provided a preparation as an orally applicable preparation by compounding with a pharmacologically active substance.

There is no particular limitation for the pharmacologically active substance so far as it is a medicine which is able to be a substrate for P-glycoprotein and is a substance which is effective when absorbed from mucous membrane of the large intestine. Its examples are medicines used for the therapy of colitis including anti-inflammatory agent such as prednisolone, sodium betamethasone phosphate, budesonide, beclomethasone dipropionate, butixocort, dexamethasone, betamethasone and fluticasone; antisense medicine; peptide or protein such as calcitonin and insulin; anti-tumor agent such as vincristine, vinblastine, doxorubicin, epirubicin, daunomycin, colchicine, actinomycin D, etoposide, teniposide, paclitaxel and cisplatin; antibiotic substance such as streptomycin, penicillin and tetracycline; and chemotherapeutic agent.

The P-glycoprotein modifier of the present invention is an agent which inhibits or promotes the action on the basis of a medicine discharge mechanism by P-glycoprotein and it may be also an agent which inhibits or promotes the expression of the P-glycoprotein. With regard to the P-glycoproteinmodifier of the present invention, there is no particular limitation so far as it has such an action.

With regard to the P-glycoprotein modifier which is compounded with the medicinal composition of the present invention, there may be exemplified verapamil, perhexilene, chlorpromazine, trifluoperazine, reserpine, amiodarone, dipyrimadole, quinidine, tamoxifen, clomiphene, cyclosporine, tacrolimus, bacinomycin, amphotericin B, bacinomycin, chloroquine, quinacrine, Tween 80 (polyoxyethylene sorbitan monooleate), valspodar and cremophor as an inhibitor while, as an enhancer, there may be exemplified adenosine triphosphate, phenothiazine, glucose, sucrose, calcium chloride and magnesium chloride.

The P-glycoprotein modifier further includes a substance which modifies the expression of CFTR (cystic fibrosis transmembrane conductance regulator) whereby the expression of P-glycoprotein is indirectly governed. As a publicly known fact, it has been known that, when CFTR is expressed on cell membrane, expression of P-glycoprotein on the cell membrane lowers while, when the expressed amount of CFTR lowers, the expressed amount of P-glycoprotein increases (*Journal of Cellular Physiology,* 161:393-406, 1994). Namely, by adding a substance inhibiting the CFTR expression on the mucous membrance cells of the large intestine, the expression of P-glycoprotein on the mucous membrance cells of the large intestine is promoted. As a result, like in the case of addition of P-glycoprotein enhancer as mentioned above, a medicine discharge system derived from P-glycoprotein is promoted and there is achieved an effect that absorption of the medicine at the upper area of the large intestine is inhibited and the absorption is targeted to the lower area of the large intestine. With regard to the CFTR expression inhibiting factor having such an action, there may be exemplified the medicines represented by HMG-CoA (3-hydroxy-3-methylglutaryl-coenzyme A) reductase inhibitor such as pravastatin, lovastatin, simvastatin and atorvastatin. On the other hand, when a substance which promotes the expression of CFTR is added, expression of P-glycoprotein is inhibited and it is possible to achieve the same effect as in the case of addition of the P-glycoprotein inhibitor.

The medicinal composition to be delivered to the large intestine according to the present invention may be a composition where the medicine and the P-glycoprotein modifier are mixed or where each of them is compounded in different layers in the preparation for administering to the large intestine. Anyway, it is sufficient that, when the part of the medicinal composition of the preparation for administering to the large intestine reaches the large intestine, the P-glycoprotein modifier according to the present invention achieves its action in the large intestine.

The preparation for administering to the large intestine according to the present invention is also characterized in that the part of the medicinal composition thereof contains the above-mentioned medicinal composition to be delivered to the large intestine according to the present invention and, as a means by which the medicinal composition of the preparation for administering to the large intestine is delivered to the large intestine, any means for the manufacture of preparation may be utilized. For example, it is possible to manufacture a preparation for administering to the large intestine having a double-coated structure in which the medicinal composition to be delivered to the large intestine of the present invention is used as a core and it is coated with an inner layer comprising a cationic copolymer and an outer layer comprising an anionic copolymer.

The compounded composition for the preparation to be delivered to the large intestine according to the present invention may be made into a preparation by compounding with appropriate filler, moisturizer, disintegrant and fluidizing agent together with the above-mentioned pharmacologically active substance and P-glycoprotein modifier. To be more specific, powder or liquid in which the pharmacologically active substance and the P-glycoprotein modifier composition are mixed is sealed in capsules or such a powder is compressed and molded followed by subjecting to coating(s) of one or more layer(s) so as to utilize as a preparation which is disintegrated in the large intestine.

For example, pharmacologically active substance which is to be a P-glycoprotein is mixed with P-glycoprotein modifier, binder, filler and disintegrant in a mixing machine such as V-shaped mixer, vertical granulator or mortar. After that, magnesium stearate is added to and mixed with the above-prepared mixture and made into tablets using an appropriate tableting machine. Then the surface of the resulting core tablets is coated with a cationic copolymer and the resulting surface is further coated with an anionic copolymer. The coating is applied by a continuous spraying of the coating solution under such a state that the said core is kept at 30°C to 50°C. An increase in weight caused by the cationic copolymer and the anionic copolymer is made 5-15% or, preferably, 6-8% of the weight of the core tablet.

The cationic polymer used as the inner layer has a property of dissolving or swelling at pH 6.0 or less. With regard to the useful polymer, there is used an aminoalkyl methacrylate copolymer (generic name) (a copolymer comprising methyl methacrylate, butyl methacrylate and dimethylaminoethyl methacrylate; trade name: Eudragit E; manufactured by Roehm) or polyvinylacetal diethylaminoacetate (trade name: AEA; manufactured by Sankyo). This polymer layer (inner layer) is used with a thickness of 10-300 µm coat thickness and with an amount of 1-40% by weight to the weight of the said solid medicine and is adjusted to such an extent that, when the condition of pH 6.0 or less continues, the active substance is quickly released from the said solid medicine. In this inner layer, an appropriate plasticizer for resulting in a smooth coating film is preferably used. The plasticizer includes triacetin, citrate ester, polyethylene glycol, etc. The binding preventer includes talc, titanium oxide, calcium phosphate, hydrophobic light silicic acid anhydride, etc.

The anionic polymer used as the outer layer has a property of easily dissolving at pH 5.5 or more. Useful polymer includes methacrylic acid copolymer L (generic name) (a copolymer comprising methacrylic acid and methyl methacrylate; trade name: Eudragit L 100; manufactured by Roehm), methacrylic acid copolymer S (generic name) (a copolymer comprising methacrylic acid and methyl methacrylate; trade name: Eudragit S; manufactured by Roehm), hydroxypropyl methylcellulose acetate succinate, hydroxypropyl methylcellulose phthalate, etc. The said polymer is used in an amount of 1-40% by weight of the said solid medicine.

When a medicinal composition wherein a pharmacologically active substance which is to be a P-glycoprotein substrate is compounded with a P-glycoprotein modifier as mentioned above is utilized as a preparation to be delivered to the large intestine, it is possible that the pharmacologically active substance is further targeted to the appropriate area in the large intestine. When the compounded P-glycoprotein modifier is a P-glycoprotein inhibitor, the medicine can be specifically targeted to the upper area of the large intestine while, when it is a P-glycoprotein enhancer, the medicine can be specifically targeted to the lower area of the large intestine. As a result, it is now possible that reduction or loss in the utilizing rate at the diseased site or the acting site in the large intestine or imbalance among individuals which are noted in the conventional preparation to be applied to the lower digestive tracts to be significantly improved.

### Examples

Compositions for administering to the large intestine compounded with the P-glycoprotein modifier of the present invention will now be illustrated in more detail and in more specific by way of the following Examples, Comparative Example and Test Example although the present invention is not limited by the following examples.

### Example 1 <Preparation of medicinal liquid compounded with sodium betamethasone phosphate and P-glycoprotein inhibitor (verapamil)>

Sodium betamethasone phosphate and verapamil were weighed according to the following formulation and dissolved in pure water.

| | |
|---|---|
| Sodium betamethasone phosphate | 2 parts by weight |
| Verapamil | 1 part by weight |

### Example 2 <Preparation of medicinal liquid compounded with sodium betamethasone phosphate and P-glycoprotein enhancer (adenosine triphosphate)>

Sodium betamethasone phosphate and adenosine triphosphate were weighed according to the following formulation and dissolved in pure water.

| | |
|---|---|
| Sodium betamethasone phosphate | 2 parts by weight |
| Adenosine triphosphate | 1 part by weight |

### Comparative Example 1 <Preparation of a sodium betamethasone phosphate solution>

Sodium betamethasone phosphate was weighed according to the following formulation and dissolved in pure water.
Sodium betamethasone phosphate 2 parts by weight

### Examples 3-8 <Preparation of tablets targeting the upper area of the large intestine>

Tablets containing a pharmacologically active substance which is to be a P-glycoprotein substrate and a P-glycoprotein inhibitor were manufactured according to the following formulations. Firstly, pharmacologically active substance, P-glycoprotein inhibitor, crystalline cellulose, lactose and crospovidone were mixed in a plastic bag and, finally, magnesium stearate was added thereto and mixed therewith. The mixture was made into tablets each having 7 mm diameter and 200 mg weight using a tableting machine.

**Table 1**

| Formulation Numbers | Ex.3 | Ex.4 | Ex.5 | Ex.6 | Ex.7 | Ex.8 |
|---|---|---|---|---|---|---|
| Na betamethasone phosphate | 2 | 2 | - | - | - | - |
| Budesonide | - | - | 2 | - | - | - |
| Doxorubicin | - | - | - | 10 | 10 | - |
| Paclitaxel | - | - | - | - | - | 10 |
| Verapamil 1 | 1 | - | 1 | 5 | - | 5 |
| Tween 80 | - | 1 | - | - | 5 | - |
| Crystalline cellulose | 10 | 10 | 10 | 10 | 10 | 10 |
| Lactose | 81 | 81 | 81 | 69 | 69 | 69 |
| Crospovidone | 5 | 5 | 5 | 5 | 5 | 5 |
| Magnesium stearate | 1 | 1 | 1 | 1 | 1 | 1 |
| * All values in the table are in part(s) by weight | | | | | | |

The resulting core was subjected to the following coating.

| | |
|---|---|
| Eudragit E | 7.0 parts by weight |
| Ethanol | 70.0 parts by weight |
| Water | 19.5 parts by weight |
| Talc | 3.5 parts by weight |

The inner layer was applied by a continuous spraying of the above solution under the state where the said core was kept at 50°C. An increase in weight of the said core was 14 mg for the tablet. After the spraying, the said core was dried and the following solution was further applied.

| | |
|---|---|
| Eudragit S | 7.0 parts by weight |
| Ethanol | 70.0 parts by weight |
| Water | 18.8 parts by weight |
| Talc | 3.5 parts by weight |
| Polyethylene glycol 6000 | 0.7 part by weight |

The outermost layer was applied by a continuous spraying of the above solution under the state where the said core was kept at 50°C. An increase in weight of the said core was 14 mg for the tablet.

### Examples 9-15 <Preparation of tablets targeting the lower area of the large intestine>

Tablets containing a pharmacologically active substance which is to be a P-glycoprotein substrate and a P-glycoprotein enhancer were manufactured according to the following formulations. Firstly, pharmacologically active substance, P-glycoprotein enhancer, crystalline cellulose, lactose and crospovidone were mixed in a plastic bag and, finally, magnesium stearate was added thereto and mixed therewith. The mixture was made into tablets each having 7 mm diameter and 200 mg weight using a tableting machine.

**Table 2**

| Formulation Numbers | Ex.9 | Ex.10 | Ex.11 | Ex.12 | Ex.13 | Ex.14 | Ex.15 |
|---|---|---|---|---|---|---|---|
| Na betamethasone phosphate | 2 | 2 | 2 | - | - | - | - |
| Budesonide | - | - | | 2 | - | - | - |
| Doxorubicin | - | - | | - | 10 | 10 | - |
| Paclitaxel | - | - | | - | - | - | 10 |
| Adenosine triphosphate | 1 | - | | 1 | 5 | - | 5 |
| Magnesium chloride | - | 1 | | - | - | 5 | - |
| Pravastatin | - | - | 1 | - | - | - | - |
| Crystalline cellulose | 10 | 10 | 10 | 10 | 10 | 10 | 10 |
| Lactose | 81 | 81 | 81 | 81 | 69 | 69 | 69 |
| Crospovidone | 5 | 5 | 5 | 5 | 5 | 5 | 5 |
| Magnesium stearate | 1 | 1 | 1 | 1 | 1 | 1 | 1 |
| * All values in the table are in part(s) by weight | | | | | | | |

The resulting core was subjected to the following coating.

| | |
|---|---|
| Eudragit E | 7.0 parts by weight |
| Ethanol | 70.0 parts by weight |
| Water | 19.5 parts by weight |
| Talc | 3.5 parts by weight |

The inner layer was applied by a continuous spraying of the above solution under the state where the said core was kept at 50°C. An increase in weight of the said core was 14 mg for the tablet. After the spraying, the said core was dried and the following solution was further applied.

| | |
|---|---|
| Eudragit S | 7.0 parts by weight |
| Ethanol | 70.0 parts by weight |
| Water | 18.8 parts by weight |
| Talc | 3.5 parts by weight |
| Polyethylene glycol 6000 | 0.7 part by weight |

The outermost layer was applied by a continuous spraying of the above solution under the state where the said core was kept at 50°C. An increase in weight of the said core was 14 mg for the tablet.

### Test Example 1

After administration of each of the medicinal solutions prepared in Examples 1 and 2 and Comparative Example 1 to the upper area of the large intestine, the residual amount of the medicine in the large intestinal lumen at the upper and the lower areas of the large intestine and the concentration of the medicine in the tissues of the large intestine were quantified and the utilizing rates of the medicine in the upper and the lower areas of the large intestine were compared.

Rats after an acclimation period for one week were fasted for 24 hours and classified into groups according to their body weights. A 20% urethane (5 ml/kg) was intraperitoneally administered to the rat, laparotomy was carried out under anesthetization and each of the medicinal solutions of Examples 1-2 and Comparative Example 1 was administered from the upper area of the large intestine at the dose of 0.25 ml per rat to the rat group each comprising three rats.

The content in the intestinal lumen and intestinal tissues were recovered after 30 minutes and 1, 3 and 6 hour(s) from the administration and the amount of the medicine remaining in the content of the intestinal lumen and the concentration of the medicine in the intestinal tissues were quantified by an HPLC.

The result is as shown in Fig. 1. Utilizing rate of sodium betamethasone phosphate in the intestinal region by each of the compounded medicine solutions was calculated. In Comparative Example 1, the utilizing rate at the upper area of the large intestine was about 40% while that at the lower area thereof was about 50%. In Example 1, the upper and the lower areas showed the utilizing rates of about 85% and about 5%, respectively. In Example 2, the upper and the lower areas showed the utilizing rates of about 5% and about 80%, respectively.

From the above result, it is now apparent that, when a P-glycoprotein modifier is compounded with the medicine solution to be administered, a targeting property to the large intestine region is significantly enhanced.

### Industrial Applicability

As fully illustrated hereinabove, the composition for administering to the large intestine compounded with a P-glycoprotein modifier according to the present invention makes a specific targeting of the medicine which is to be a P-glycoprotein substrate to the appropriate area of the large intestine (upper or lower area) possible.

## Claims

1. A medicinal composition to be delivered to the large intestine, **characterized in that**, a P-glycoprotein modifier is compounded therewith.

2. The medicinal composition to be delivered to the large intestine according to claim 1, wherein a pharmacologically active substance which is to be a substrate for P-glycoprotein is compounded with a P-glycoprotein modifier.

3. The medicinal composition to be delivered to the large intestine according to claim 1 or 2, wherein the P-glycoprotein modifier is a P-glycoprotein inhibitor or a P-glycoprotein enhancer.

4. The medicinal composition to be delivered to the large intestine according to claim 3, wherein the P-glycoprotein inhibitor is verapamil, perhexilene, chlorpromazine, trifluoperazine, reserpine, amiodarone, dipyrimadole, quinidine, tamoxifen, clomiphene, cyclosporine, tacrolimus, bacinomycin, amphotericin B, bacinomycin, chloroquine, quinacrine, Tween 80 (polyoxyethylene sorbitan monooleate), valspodar or cremophor.

5. The medicinal composition to be delivered to the large intestine according to claim 3, wherein the P-glycoprotein enhancer is adenosine triphosphate, phenothiazine, glucose, sucrose, calcium chloride or magnesium chloride.

6. The medicinal composition to be delivered to the large intestine according to claim 3, wherein the P-glycoprotein modifier is an expression regulating factor for CFTR (cystic fibrosis transmembrane conductance regulator).

7. The medicinal composition to be delivered to the large intestine according to claim 3 or 6, wherein the P-glycoprotein enhancer is an expression inhibitor for CFTR (cystic fibrosis transmembrane conductance regulator).

8. The medicinal composition to be delivered to the large intestine according to claim 7, wherein the expression inhibitor for CFTR (cystic fibrosis transmembrane conductance regulator) is HMG-CoA (3-hydroxy-3-methylglutaryl-coenzyme A) reductase inhibitor.

9. The medicinal composition to be delivered to the large intestine according to claim 8, wherein the HMG-CoA reductase inhibitor is pravastatin, lovastatin, simyastatin, atorvastatin, cerivastatin, fluvastatin or itavastatin.

10. The medicinal composition to be delivered to the large intestine according to claims 1 to 9, wherein the compounding ratio of the pharmacologically active substance to the P-glycoprotein modifier is from 1:0.01 to 1:10.

11. The medicinal composition to be delivered to the large intestine according to claims 1 to 10, wherein the pharmacologically active substance which is to be a substrate for the P-glycoprotein is steroidal medicine, anti-steroidal anti-inflammatory agent, antisense medicine, peptides, anti-tumor medicine, antibiotic substance and chemotherapeutic medicine.

12. Apreparation for administering to the large intestine containing the medicinal composition to be delivered to the large intestine mentioned in any of claims 1 to 11.
